# EUROPEAN PATENT APPLICATION

(11) **EP 3 763 220 A1**
(43) Date of publication of application: **13.01.2021**
(21) Application number: 20175806.7
(22) Date of filing: 13.02.2008
(51) Int. Cl.: A23C 19/04, C12N 9/64

(54) **COAGULATION OF MILK**

(30) Priority: 13.02.2007 US 900978 P; 21.02.2007 EP 07102829
(62) Divisional of application: 08708967.8
(71) Applicant: Chr. Hansen A/S, 2970 Hørsholm (DK)
(72) Inventor: BROE, Mikkel Laust, 2720 Vanloese (DK); VAN DEN BRINK, Johannes Marten, 2730 Herlev (DK); HARBOE, Marianne Kirsten, 2800 Lyngby (DK)
(74) Representative: Uexküll & Stolberg

(57) **Abstract**

The present invention relates to methods for coagulating bovine milk and making cheese using a coagulant substantially identical to a chymosin originating from an animal of the *Tylopoda* suborder.

SEQUENCE LISTING

<110>  Chr. Hansen A/S

<120>  Coagulation of milk

<130>  P2922PC00

<140>  EP 07102829.4
<141>  2007-02-21

<160>  11

<170>  PatentIn version 3.3

<210>  1
<211>  381
<212>  PRT
<213>  Camelus dromedarius

<400>  1

Met Arg Cys Leu Val Val Leu Leu Ala Ala Leu Ala Leu Ser Gln Ala
1               5                   10                  15

Ser Gly Ile Thr Arg Ile Pro Leu His Lys Gly Lys Thr Leu Arg Lys
            20                  25                  30

Ala Leu Lys Glu Arg Gly Leu Leu Glu Asp Phe Leu Gln Arg Gln Gln
        35                  40                  45

Tyr Ala Val Ser Ser Lys Tyr Ser Ser Leu Gly Lys Val Ala Arg Glu
    50                  55                  60

Pro Leu Thr Ser Tyr Leu Asp Ser Gln Tyr Phe Gly Lys Ile Tyr Ile
65                  70                  75                  80

Gly Thr Pro Pro Gln Glu Phe Thr Val Val Phe Asp Thr Gly Ser Ser
                85                  90                  95

Asp Leu Trp Val Pro Ser Ile Tyr Cys Lys Ser Asn Val Cys Lys Asn
            100                 105                 110

His His Arg Phe Asp Pro Arg Lys Ser Ser Thr Phe Arg Asn Leu Gly
        115                 120                 125

Lys Pro Leu Ser Ile His Tyr Gly Thr Gly Ser Met Glu Gly Phe Leu
    130                 135                 140

Gly Tyr Asp Thr Val Thr Val Ser Asn Ile Val Asp Pro Asn Gln Thr
145                 150                 155                 160

Val Gly Leu Ser Thr Glu Gln Pro Gly Glu Val Phe Thr Tyr Ser Glu

                165                 170                 175

Phe Asp Gly Ile Leu Gly Leu Ala Tyr Pro Ser Leu Ala Ser Glu Tyr
            180                 185                 190

Ser Val Pro Val Phe Asp Asn Met Met Asp Arg His Leu Val Ala Arg
        195                 200                 205

Asp Leu Phe Ser Val Tyr Met Asp Arg Asn Gly Gln Gly Ser Met Leu
    210                 215                 220

Thr Leu Gly Ala Ile Asp Pro Ser Tyr Tyr Thr Gly Ser Leu His Trp
225                 230                 235                 240

Val Pro Val Thr Leu Gln Gln Tyr Trp Gln Phe Thr Val Asp Ser Val
                245                 250                 255

Thr Ile Asn Gly Val Ala Val Ala Cys Val Gly Gly Cys Gln Ala Ile
            260                 265                 270

Leu Asp Thr Gly Thr Ser Val Leu Phe Gly Pro Ser Ser Asp Ile Leu
        275                 280                 285

Lys Ile Gln Met Ala Ile Gly Ala Thr Glu Asn Arg Tyr Gly Glu Phe
    290                 295                 300

Asp Val Asn Cys Gly Asn Leu Arg Ser Met Pro Thr Val Val Phe Glu
305                 310                 315                 320

Ile Asn Gly Arg Asp Tyr Pro Leu Ser Pro Ser Ala Tyr Thr Ser Lys
                325                 330                 335

Asp Gln Gly Phe Cys Thr Ser Gly Phe Gln Gly Asp Asn Asn Ser Glu
            340                 345                 350

Leu Trp Ile Leu Gly Asp Val Phe Ile Arg Glu Tyr Tyr Ser Val Phe
        355                 360                 365

Asp Arg Ala Asn Asn Arg Val Gly Leu Ala Lys Ala Ile
    370                 375                 380

<210> 2
<211> 381
<212> PRT
<213> Camelus bactrianus

<400> 2

Met Arg Cys Leu Val Val Leu Leu Ala Ala Leu Ala Leu Ser Gln Ala

1               5                   10                  15

Ser Gly Ile Thr Arg Ile Pro Leu His Lys Gly Lys Thr Leu Arg Lys
            20                  25                  30

Ala Leu Lys Glu Arg Gly Leu Leu Glu Asp Phe Leu Gln Arg Gln Gln
        35                  40                  45

Tyr Ala Val Ser Ser Lys Tyr Ser Ser Leu Gly Lys Val Ala Arg Glu
    50                  55                  60

Pro Leu Thr Ser Tyr Leu Asp Ser Gln Tyr Phe Gly Lys Ile Tyr Ile
65                  70                  75                  80

Gly Thr Pro Pro Gln Glu Phe Thr Val Val Phe Asp Thr Gly Ser Ser
                85                  90                  95

Asp Leu Trp Val Pro Ser Ile Tyr Cys Lys Ser Asn Ala Cys Lys Asn
            100                 105                 110

His His Arg Phe Asp Pro Arg Lys Ser Ser Thr Phe Arg Asn Leu Gly
        115                 120                 125

Lys Pro Leu Ser Ile His Tyr Gly Thr Gly Ser Ile Glu Gly Phe Leu
    130                 135                 140

Gly Tyr Asp Thr Val Thr Val Ser Asn Ile Val Asp Pro Asn Gln Thr
145                 150                 155                 160

Val Gly Leu Ser Thr Glu Gln Pro Gly Glu Val Phe Thr Tyr Ser Glu
                165                 170                 175

Phe Asp Gly Ile Leu Gly Leu Ala Tyr Pro Ser Leu Ala Ser Glu Tyr
            180                 185                 190

Ser Val Pro Val Phe Asp Asn Met Met Asp Arg His Leu Val Ala Arg
        195                 200                 205

Asp Leu Phe Ser Val Tyr Met Asp Arg Asn Gly Gln Gly Ser Met Leu
    210                 215                 220

Thr Leu Gly Ala Thr Asp Pro Ser Tyr Tyr Thr Gly Ser Leu His Trp
225                 230                 235                 240

Val Pro Val Thr Val Gln Gln Tyr Trp Gln Val Thr Val Asp Ser Val
                245                 250                 255

Thr Ile Asn Gly Val Ala Val Ala Cys Val Gly Gly Cys Gln Ala Ile
            260                 265                 270

Leu Asp Thr Gly Thr Ser Val Leu Phe Gly Pro Ser Ser Asp Ile Leu
        275                 280                 285

Lys Ile Gln Met Ala Ile Gly Ala Thr Glu Asn Arg Tyr Gly Glu Phe
    290                 295                 300

Asp Val Asn Cys Gly Ser Leu Arg Ser Met Pro Thr Val Val Phe Glu
305                 310                 315                 320

Ile Asn Gly Arg Asp Phe Pro Leu Ala Pro Ser Ala Tyr Thr Ser Lys
                325                 330                 335

Asp Gln Gly Phe Cys Thr Ser Gly Phe Gln Gly Asp Asn Asn Ser Glu
            340                 345                 350

Leu Trp Ile Leu Gly Asp Val Phe Ile Arg Glu Tyr Tyr Ser Val Phe
        355                 360                 365

Asp Arg Ala Asn Asn Arg Val Gly Leu Ala Lys Ala Ile
    370                 375                 380

<210>  3
<211>  381
<212>  PRT
<213>  Lama glama

<400>  3

Met Arg Cys Leu Val Val Leu Leu Ala Ala Leu Ala Leu Ser Gln Ala
1               5                   10                  15

Ser Gly Ile Thr Arg Ile Pro Leu Tyr Lys Gly Lys Thr Leu Arg Lys
            20                  25                  30

Ala Leu Lys Glu His Gly Leu Leu Glu Asp Phe Leu Gln Arg Gln Gln
        35                  40                  45

Tyr Ala Val Ser Ser Lys Tyr Ser Ser Leu Gly Lys Val Ala Arg Glu
    50                  55                  60

Pro Leu Thr Ser Tyr Leu Asp Ser Gln Tyr Phe Gly Lys Ile Tyr Ile
65                  70                  75                  80

Gly Thr Pro Pro Gln Glu Phe Thr Val Val Phe Asp Thr Gly Ser Ser
                85                  90                  95

Asp Leu Trp Val Pro Ser Ile Tyr Cys Lys Ser Asn Val Cys Lys Asn
        100                 105                 110

His His Arg Phe Asp Pro Arg Lys Ser Ser Thr Phe Arg Asn Leu Gly
    115                 120                 125

Lys Pro Leu Ser Ile His Tyr Gly Thr Gly Ser Met Glu Gly Phe Leu
130                 135                 140

Gly Tyr Asp Thr Val Thr Val Ser Asn Ile Val Asp Pro Asn Gln Thr
145                 150                 155                 160

Val Gly Leu Ser Thr Glu Gln Pro Gly Glu Val Phe Thr Tyr Ser Glu
                165                 170                 175

Phe Asp Gly Asn Leu Gly Leu Ala Tyr Pro Ser Leu Ala Ser Glu Tyr
            180                 185                 190

Ser Val Pro Val Phe Asp Asn Met Met Asp Arg His Leu Val Ala Gln
        195                 200                 205

Asp Leu Phe Ser Val Tyr Met Asp Arg Asn Gly Gln Gly Ser Met Leu
    210                 215                 220

Thr Leu Gly Ala Ile Asp Ser Ser Tyr Tyr Thr Gly Ser Leu His Trp
225                 230                 235                 240

Val Pro Val Thr Val Gln Gln Tyr Trp Gln Val Thr Val Asp Ser Val
                245                 250                 255

Thr Ile Asn Gly Val Ala Val Ala Cys Val Gly Gly Cys Gln Ala Ile
            260                 265                 270

Leu Asp Thr Gly Thr Ser Val Leu Phe Gly Pro Ser Ser Asp Ile Leu
        275                 280                 285

Lys Ile Gln Lys Ala Ile Gly Ala Thr Glu Asn Arg Tyr Gly Glu Phe
    290                 295                 300

Asp Val Asn Cys Gly Asn Leu Arg Ser Met Pro Thr Val Val Phe Glu
305                 310                 315                 320

Ile Asn Gly Arg Asp Tyr Pro Leu Ser Pro Ser Ala Tyr Thr Ser Lys
                325                 330                 335

Asp Gln Gly Phe Cys Thr Ser Gly Phe Gln Gly Asp Asn Asn Ser Glu
            340                 345                 350

Leu Trp Ile Leu Gly Asp Val Phe Ile Arg Glu Tyr Tyr Ser Val Phe
        355                 360                 365

Asp Arg Ala Asn Asn Arg Val Gly Leu Ala Lys Ala Ile
    370                 375                 380

<210> 4
<211> 323
<212> PRT
<213> Lama guanicoe

<220>
<221> misc_feature
<222> (53)..(92)
<223> Xaa can be any naturally occurring amino acid

<220>
<221> misc_feature
<222> (159)..(197)
<223> Xaa can be any naturally occurring amino acid

<400> 4

Gly Lys Val Ala Arg Glu Pro Leu Thr Ser Tyr Leu Asp Ser Gln Tyr
1               5                   10                  15

Phe Gly Lys Ile Tyr Ile Gly Thr Pro Pro Gln Glu Phe Thr Val Val
            20                  25                  30

Phe Asp Thr Gly Ser Ser Asp Leu Trp Val Pro Ser Ile Tyr Cys Lys
        35                  40                  45

Ser Asn Ala Cys Xaa Xaa Xaa Xaa Xaa Xaa Xaa Xaa Xaa Xaa Xaa Xaa
    50                  55                  60

Xaa Xaa Xaa Xaa Xaa Xaa Xaa Xaa Xaa Xaa Xaa Xaa Xaa Xaa Xaa Xaa
65                  70                  75                  80

Xaa Xaa Xaa Xaa Xaa Xaa Xaa Xaa Xaa Xaa Xaa Xaa Val Ser Asn Ile
                85                  90                  95

Val Asp Pro Asn Gln Thr Val Gly Leu Ser Thr Glu Gln Pro Gly Glu
            100                 105                 110

Val Phe Thr Tyr Ser Glu Phe Asp Gly Ile Leu Gly Leu Ala Tyr Pro
        115                 120                 125

Ser Leu Ala Ser Glu Tyr Ser Val Pro Val Phe Asp Asn Met Met Asp
    130                 135                 140

Arg His Leu Val Ala Gln Asp Leu Phe Ser Val Tyr Met Asp Xaa Xaa
145                 150                 155                 160

Xaa Xaa Xaa Xaa Xaa Xaa Xaa Xaa Xaa Xaa Xaa Xaa Xaa Xaa Xaa Xaa
                165                 170                 175

Xaa Xaa Xaa Xaa Xaa Xaa Xaa Xaa Xaa Xaa Xaa Xaa Xaa Xaa Xaa Xaa
            180                 185                 190

Xaa Xaa Xaa Xaa Xaa Val Thr Ile Asn Gly Val Ala Val Ala Cys Val
        195                 200                 205

Gly Gly Cys Gln Ala Ile Leu Asp Thr Gly Thr Ser Val Leu Phe Gly
    210                 215                 220

Pro Ser Ser Asp Ile Leu Lys Ile Gln Met Ala Ile Gly Ala Thr Glu
225                 230                 235                 240

Asn Arg Tyr Gly Glu Phe Asp Val Asn Cys Gly Asn Leu Arg Ser Met
                245                 250                 255

Pro Thr Val Val Phe Glu Ile Asn Gly Arg Asp Phe Pro Leu Ala Pro
            260                 265                 270

Ser Ala Tyr Thr Ser Lys Asp Gln Gly Phe Cys Thr Ser Gly Phe Gln
        275                 280                 285

Ser Glu Asn His Ser Gln Lys Trp Ile Leu Gly Asp Val Phe Ile Arg
    290                 295                 300

Glu Tyr Tyr Ser Val Phe Asp Arg Ala Asn Asn Leu Val Gly Leu Ala
305                 310                 315                 320

Lys Ala Ile

<210>  5
<211>  30
<212>  PRT
<213>  Camelus bactrianus

<400>  5

Tyr Gly Glu Phe Asp Val Asn Cys Gly Ser Leu Arg Ser Met Pro Thr
1               5                   10                  15

Val Val Phe Glu Ile Asn Gly Arg Asp Phe Pro Leu Ala Pro
            20                  25                  30

<210>  6
<211>  30
<212>  PRT
<213>  Camelus dromedarius

<400>  6

Tyr Gly Glu Phe Asp Val Asn Cys Gly Asn Leu Arg Ser Met Pro Thr
1               5                   10                  15

Val Val Phe Glu Ile Asn Gly Arg Asp Tyr Pro Leu Ser Pro
            20                  25                  30

<210>  7
<211>  30
<212>  PRT
<213>  Lama glama

<400>  7

Tyr Gly Glu Phe Asp Val Asn Cys Gly Asn Leu Arg Ser Met Pro Thr
1               5                   10                  15

Val Val Phe Glu Ile Asn Gly Arg Asp Tyr Pro Leu Ser Pro
            20                  25                  30

<210>  8
<211>  1133
<212>  DNA
<213>  Camelus dromedarius

<400>  8
gacggtgact gacacgtggc ggagtgggat caccaggatc cctctgcaca aaggcaagac      60

tctgagaaaa gcgctgaagg agcgtgggct cctggaggac tttctgcaga gacaacagta     120

tgccgtcagc agcaagtact ccagcttggg gaaggtggcc agggaacccc tgaccagcta     180

cctggatagt cagtactttg ggaagatcta catcgggacc ccaccccagg agttcaccgt     240

ggtgtttgac actggctcct ctgacctgtg ggtgccctct atctactgca agagcaatgt     300

ctgcaaaaac caccaccgct ttgacccgag aaagtcgtcc accttccgga acctgggcaa     360

gcccctgtcc atccattacg gcacgggcag catggagggc tttctgggct acgacaccgt     420

caccgtctcc aacattgtgg accccaacca gactgtgggc ctgagcaccg agcaacctgg     480

cgaggtcttc acctactccg agtttgacgg gatcctgggg ctggcctacc cctcgcttgc     540

ctccgagtac tcggtgcccg tgtttgacaa tatgatggac agacacctgg tggcccgaga     600

cctgttctcg gtttacatgg acaggaatgg ccaggggagc atgcttacac tgggggccat     660

tgacccgtcc tactacaccg gctccctgca ctgggtgccc gtgaccttgc agcagtactg     720

gcagttcacc gtggacagtg tcaccatcaa cggggtggca gtggcctgtg ttggtggctg     780

tcaggccatc ctggacacgg gtacctccgt gctgttcggg cccagcagcg acatcctcaa     840

aattcagatg gctattggag ccacagagaa ccgatatggt gagtttgacg tcaactgtgg 900

gaacctgagg agcatgccca ccgtggtctt cgagatcaat ggcagagact acccactgtc 960

cccctccgcc tacacaagca aggaccaggg cttctgcacc agtggctttc aaggtgacaa 1020

caattccgag ctgtggatcc tgggggatgt cttcatccgg gagtattaca gtgtctttga 1080

cagggccaac aatcgcgtgg ggctggccaa ggccatctga tcctctagag tcg 1133

<210> 9
<211> 1159
<212> DNA
<213> Camelus bactrianus

<400> 9
atgcggtgcc tcgtggtgct acttgcagcc ctcgctctct cccaggccag tgggatcacc 60

aggatccctc tgcacaaagg caagactctg agaaaagcgc tgaaggagcg tgggctcctg 120

gaggactttc tgcagagaca acagtatgcc gtcagcagca agtactccag cttggggaag 180

gtggccaggg aacccctgac cagctacctg gatagtcagt actttgggaa gatctacatc 240

gggaccccac cccaggagtt caccgtggtg tttgacactg gctcctctga cctgtgggtg 300

ccctctatct actgcaagag caatgcctgc aaaaaccacc accgctttga cccgagaaag 360

tcgtccacct tccggaacct gggcaagccc ctgtccatcc attacggcac gggcagcatt 420

gagggctttc tgggctacga caccgtcacc gtctccaaca ttgtggaccc caaccagact 480

gtgggcctga gcaccgagca acctggcgag gtcttcacct actccgagtt tgacgggatc 540

ctggggctgg cctacccctc gcttgcctcc gagtactcgg tgcccgtgtt tgacaatatg 600

atggacagac acctggtggc ccgagacctg ttctcggttt acatggacag gaatggccag 660

gggagcatgc ttacactggg ggccactgac ccctcctact acaccggctc cctgcactgg 720

gtgcccgtga ccgtgcagca gtactggcag gtcaccgtgg acagtgtcac catcaacggg 780

gtggcagtgg cctgtgttgg tggctgtcag gccatcctgg acacgggtac ctccgtgctg 840

ttcgggccca gcagcgacat cctcaaaatt cagatggcta ttggagccac agagaaccga 900

tatggtgagt ttgacgtcaa ctgtgggagc ctgaggagca tgcccaccgt ggtcttcgag 960

atcaatggca gagacttccc actggccccc tccgcctaca caagcaagga ccagggcttc 1020

tgcaccagtg gctttcaagg tgacaacaat tccgagctgt ggatcctggg ggatgtcttc 1080

atccgggagt attacagtgt ctttgacagg gccaacaatc gcgtggggct ggccaaggcc 1140

atctgatcct ctagagtcg 1159

<210> 10
<211> 1143
<212> DNA
<213> Lama glama

<400>  10
acgtcgaccg cgacggtgac tgacacgtgg cgtgccgaga tcactcgcat ccccctctac      60

aagggcaagc ctctgcgtaa ggctctcaag gagcgcggtc tgctcgagga tttcctgcag      120

aagcagcagt acggcgtcag ctctgagtac agcggtttcg gcgaggtggc cagcgtgcct      180

ctcactaact acctggacag ccagtacttc ggtaagatct accttggcac tccccctcag      240

gagttcaccg ttctgttcga tactggttcc agcgacttct gggttccctc catctactgt      300

aagagcaacg cttgcaagaa ccaccagcgc ttcgatcctc gcaagtccag caccttccag      360

aaccttggca agcccctttc catccgctac ggtactggca gcatgcaggg tatccttggc      420

tacgacaccg ttaccgtgtc caacatcgtc gatattcagc agaccgtggg tctgagcacc      480

caggagcctg gcgatgtctt cacttacgcc gagttcgatg gtatcctcgg catggcttac      540

ccctccctgg cctctgagta ctctgtccct gtgttcgaca acatgatgga ccgccgcctc      600

gtcgctcagg atctgttcag cgtgtacatg gaccgtagcg gtcaggggtc catgcttact      660

ctgggcgcca tcgatccctc ttactacacc ggttccctcc actgggttcc tgtgaccctc      720

cagaagtact ggcagttcac cgtggacagc gtcactatct ccggcgcggt tgtggcttgc      780

gagggtggct gtcaggccat ccttgatact ggtaccagca agctcgtcgg cccctccagc      840

gacatcctga acatccagca ggctatcggt gccacccaga accagtacgg cgagttcgat      900

atcgactgcg atagcctttc ttccatgcct actgtggttt tcgagatcaa cggtaagatg      960

taccccctta ctccttatgc ttacacttcc caggaggagg gcttctgtac ctctggtttc     1020

cagggtgaga accacagcca ccagtggatc cttggcgatg tcttcatccg cgagtactac     1080

tccgtcttcg accgtgccaa caacctggtg ggtctcgcta aggccatctg atcctctaga     1140

gtc                                                                   1143

<210>  11
<211>  1098
<212>  DNA
<213>  Lama guanicoe

<220>
<221>  misc_feature
<222>  (1)..(2)
<223>  n is a, c, g, or t

<220>
<221>  misc_feature
<222>  (276)..(402)
<223>  n is a, c, g, or t

<220>
<221>  misc_feature
<222>  (603)..(716)
<223>  n is a, c, g, or t

<400>  11
nncttgatcc ctctgtacaa aggcaagact ctgagaaaag cgttgaagga gcatgggctc      60

ctggaggact ttctgcagag acaacagtat gccgtcagca gcaagtactc cagcttgggg     120

aaggtggcca gggaacccct gaccagctac ctggatagtc agtactttgg gaagatctac     180

atcgggaccc caccccagga gttcaccgtg gtgtttgaca ctggctcctc cgacctgtgg     240

gtgccctcta tctactgcaa gagcaatgcc tgcaannnnn nnnnnnnnnn nnnnnnnnnn     300

nnnnnnnnnn nnnnnnnnnn nnnnnnnnnn nnnnnnnnnn nnnnnnnnnn nnnnnnnnnn     360

nnnnnnnnnn nnnnnnnnnn nnnnnnnnnn nnnnnnnnnn nngtctccaa cattgtggac     420

cccaaccaga ctgtgggcct gagcaccgag caacctggcg aggtcttcac ctactccgaa     480

tttgacggga tcctggggct ggcctacccc tcgcttgcct ccgagtactc ggtgcccgtg     540

tttgacaata tgatggacag acacctggtg gcccaagacc tgttctcggt ttacatggac     600

agnnnnnnnn nnnnnnnnnn nnnnnnnnnn nnnnnnnnnn nnnnnnnnnn nnnnnnnnnn     660

nnnnnnnnnn nnnnnnnnnn nnnnnnnnnn nnnnnnnnnn nnnnnnnnnn nnnnnntgtc     720

accatcaacg gggtggcagt ggcctgtgtt ggtggctgtc aggccatcct ggacacgggt     780

acctccgtgc tgttcggccc cagcagcgac atcctcaaaa ttcagatggc tattggagcc     840

acagagaacc gatatggtga gtttgacgtc aactgtggga acctgaggag catgcccacc     900

gtggtcttcg agatcaatgg cagagacttc ccactggccc cctccgccta cacaagcaag     960

gaccagggct tctgcaccag tggcttccag agtgaaaatc attcccagaa atggatcctg    1020

ggggatgttt tcatccgaga gtattacagc gtctttgaca gggccaacaa cctcgtgggg    1080

ctggccaagg ccatctga                                                  1098

## Description

### FIELD OF INVENTION

The present invention relates to methods for coagulating bovine milk and making a dairy product such as cheese using a coagulant identical or substantially identical to a chymosin originating from an animal of the *Tylopoda* suborder.

### BACKGROUND OF INVENTION

Methods for coagulating milk have been known for centuries, the most important method is coagulating cow's milk by contacting the milk with chymosin originating from the abomasum of a calf, or with a chymosin that has the same amino acid sequence, but being produced using recombinant cells.

WO 02/36752 A2 discloses a method for clotting bovine skimmed milk using 3.1 nM recombinant camel chymosin (example 5); coagulation of reconstituted bovine skimmed milk and raw cow's milk using 65 IMCU/I (example 6 and 7, resp); and whole pasteurized milk using 35 IMCU/I . It is stated that the concentration of 3.1 nM was equivalent in clotting activity (measured in IMCU's) to 5.4 nM bovine chymosin, and that camel chymosin is less affected by changes in pH and Ca2+ concentration.

In Journal of Dairy Research (2000) 67 73-81, E. I. Elagamy states that extracts of camel abomasum (camel rennet comprising chymosin and pepsin) have been used to coagulate cow's milk.

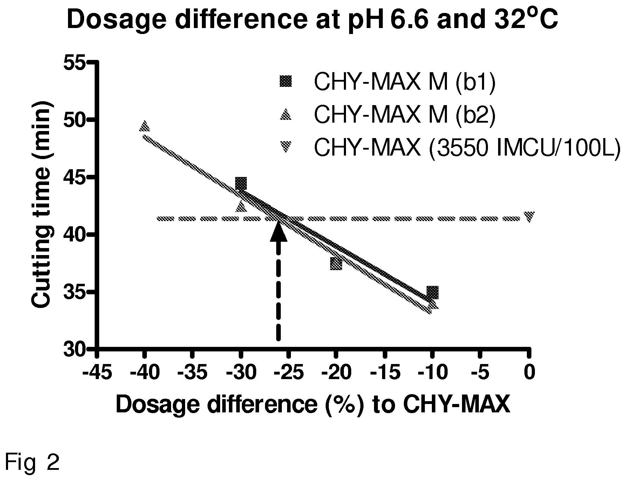

Wangoh et al, Milchwissenschaft (1993) 48, 322 discloses that camel rennet (abomasum extract comprising chymosin and pepsin) is able to coagulate cow's milk. Coagulation of the milk seems to have been carried out at a pH below 4.7, see figure 2.

### SUMMARY OF INVENTION

The present invention is based on the surprising finding that camel chymosin - besides a 70% higher specific activity - additionally gives a 20% faster curd formation (time to cutting) than bovine chymosin does under the same conditions, ie the same amount in IMCU's is used. This means that below 40% of the mg needed for calf chymosin B is needed of camel chymosin for the same application in a typical cheese manufacture.

The milk coagulation process to form a curd may be considered as a two-phase sequence:
1) a first phase in which kappa-casein is hydrolyzed. This phase is measured by the formation of visible flocculation (also called clotting).
2) a second phase in which the flocks is aggregated to form a 3-dimentional gel/network. This phase is measured by the formation of curd firmness.

The strength (activity) of a chymosin enzyme preparation is found by measuring its milk clotting activity relative to an international enzyme standard with known activity (measured in the period from addition of the enzyme to a milk until formation of visible flocks or flakes in the milk). A measure of the strength is the International Milk Clotting Unit per volume or weight (eg. IMCU/ml).

The present inventors have observed from curd formation trials in laboratory as well as in cheese production, using bovine milk, that a lower amount of camel chymosin (measured in International Milk Clotting Units (IMCU)) is necessary compared to bovine calf chymosin in order to obtain the same curd firmness under same conditions. Or said differently, if same dosage in IMCU is used then the curd will form faster when camel chymosin is used instead of bovine chymosin. More specifically, the present inventors have surprisingly found out that contacting bovine milk with a recombinantly produced camel chymosin enzyme results in a substantially faster setting time compared to when using a camel rennet (which besides chymosin comprises pepsin) and/or compared to when using recombinantly produced bovine chymosin. When the pH of the bovine milk is 6.5, the amount of camel chymosin (IMCU) can be reduced about 20% compared the amount of bovine chymosin (IMCU) necessary for coagulating the milk under the same conditions - or the time for coagulating the milk can be reduced correspondently. This finding is contrary to the findings in WO 02/36752.

Without wishing to be bound to any theory, it is presently contemplated that the flocks aggregate faster, i.e. the second phase is shorter in time, when milk is treated with camel chymosin compared to when milk is treated with bovine chymosin, using the same amount of IMCU.

In accordance with this finding, the present invention in a presently preferred aspect pertains to a process for making a curd by contacting bovine milk with a chymosin enzyme originating from a camel, said chymosin is added in an amount not exceeding 30 IMCU per liter milk and/or said chymosin is added in an amount not exceeding 90% of the amount (measured in IMCU) of bovine chymosin B that would have been necessary for obtaining a curd with the same strength in the same time and at the same temperature using the same milk.

In an other aspect, the present invention pertains to a process for decreasing the time for making a cheese, and to a process for obtaining cheese in high yield (higher cheese yield (dry matter yield) compared to bovine chymosin B), the processes comprise contacting a milk with a chymosin enzyme originating from a camel.

In addition, the present inventors have found out that cheese based on cows milk coagulated with recombinantly produced camel chymosin has several unexpected differences from cheese based on cows milk coagulated with bovine chymosin (CHY-MAX ®), such as:
- a pleasant taste and flavor (reduced bitterness, reduced sulphur flavor and reduced brothy flavor); and
- a good texture (better mouth feel, less breakdown, less smoothness, less cohesive and less adhesive).

In accordance with these surprising findings the invention relates to a method for improving taste and/or texture of cheese, comprising contacting bovine milk with a chymosin enzyme having an amino acid sequence identical or substantially identical to the amino acid sequence of chymosin (EC 3.4.23.4) from an animal of the suborder *Tylopoda.* The curd obtained can be further processed to cheese in a manner known to the skilled person.

### DETAILED DISCLOSURE

The present invention relates to method for coagulation of milk (or aggregation of casein micelles), comprising contacting bovine milk with a chymosin enzyme having an amino acid sequence identical or substantially identical to the amino acid sequence of chymosin (EC 3.4.23.4) from an animal of the suborder *Tylopoda.* It is presently preferred that said chymosin is added in an amount not exceeding 30 IMCU per liter milk and/or said chymosin is added in an amount not exceeding 90% of the amount (measured in IMCU) of bovine chymosin B (such as CHY-MAX ®) that would have been necessary for obtaining a curd with the same strength under comparative conditions (in the same time and at the same temperature using the same milk).

The coagulated milk may be drained of the liquid portion (called whey) for obtaining a curd, and therefore the present invention also relates to a method for producing a curd, comprising contacting bovine milk with a chymosin enzyme having an amino acid sequence identical or substantially identical to the amino acid sequence of chymosin (EC 3.4.23.4) from an animal of the suborder *Tylopoda.* It is presently preferred that said chymosin is added in an amount not exceeding 30 IMCU per liter milk and/or said chymosin is added in an amount not exceeding 90% of the amount (measured in IMCU) of bovine chymosin B that would have been necessary for obtaining a curd with the same strength in the same time and at the same temperature using the same milk.

The curd may be further processed to obtain cheese. Therefore, the present invention also relates to a method for manufacturing cheese (such as cheese having an good texture and/or taste), comprising contacting bovine milk with a chymosin enzyme having an amino acid sequence identical or substantially identical to the amino acid sequence of chymosin (EC 3.4.23.4) from an animal of the suborder *Tylopoda.* It is presently preferred that said chymosin is added in an amount not exceeding 30 IMCU per liter milk and/or said chymosin is added in an amount not exceeding 90% of the amount (measured in IMCU) of bovine chymosin B that would have been necessary for obtaining a curd with the same strength in the same time and at the same temperature using the same milk.

In a second aspect, the present invention relates to a method for improving taste and/or texture of cheese, comprising contacting bovine milk with a chymosin enzyme having an amino acid sequence identical or substantially identical to the amino acid sequence of chymosin (EC 3.4.23.4) from an animal of the suborder *Tylopoda.* In a presently preferred embodiment, said chymosin is added in an amount not exceeding 30 IMCU per liter milk and/or said chymosin is added in an amount not exceeding 90% of the amount (measured in IMCU) of bovine chymosin B that would have been necessary for obtaining a curd with the same strength under comparative conditions.

In the above methods, it is presently preferred the chymosin enzyme is used in an amount below 28, such as below 26, below 24, below 22 or even below 20 IMCU per liter milk. The enzyme may be used in the ratio from 5 to 25 IMCU per liter of milk, e.g. 10-22 IMCU per liter, or 4.8 mg pure enzyme pr 100 liter of milk. In another embodiment, the chymosin is added to the milk in an amount not exceeding 90% (such as not exceeding 88%, not exceeding 85%, not exceeding 83%, not exceeding 80%, not exceeding 78%, not exceeding 75%, or even not exceeding 73%, 70% or 65%) of the amount (measured in IMCU) of bovine chymosin B (such as CHY-MAX tm) that would have been necessary for obtaining a curd with the same strength in the same time and at the same temperature using the same milk.

Instead of using a lower amount of coagulant, the coagulating process may be accelerated (a curd with the desired firmness can be obtained faster) by using the same amount of the chymosin enzyme according to the invention instead of bovine chymosin B (measured in IMCU). Therefore, the present invention also relates to a method for obtaining a curd (such as with a firmness of 20mm +/- 5mm (measured on Formagraph equipment)), comprising contacting cows milk, preferably having a pH within the range 6.3 to 6.7 and preferable having a temperature within the range 31-33 degrees C, with *Tylopoda* chymosin at a concentration below 950 IMCU multiplied with the volume of milk in liter and divided by the desired time for coagulation (cutting time) in minutes (i.e. calculated as 950 x L / minutes). In a preferred embodiment, the chymosin is used at a concentration below 930 IMCU x L / minutes, such as below 910, below 880, below 850, below 820 or even below 790 or 700 IMCU x L / minutes. It is presently preferred that the concentration is in the range 840 to 920 IMCU x L milk / minutes, but other concentrations may be used, dependent on the desired curd strength/firmness, the milk used, and the temperature. For instance, if a more firm curd is desired, the range may be 850 to 1000 IMCU x L / minutes, 900-1100, or even 1000 to 1300 IMCU x L / minutes, or if a lesser firm curd is desired, the range may be 800 to 900 IMCU x L / minutes, or even lower.

Such variations - which may easily be calculated using the data in this document - are embodiments of the present invention.

The curd obtained by any of the above methods may be further processed for manufacturing cheese by treating the curd in a manner known per se and described in the literature (e.g. "Cheese and Fermented Milk Foods" by Frank V. Kosikowski and Vikram V. Mistry). The resulting cheese may be a cheese selected from the group consisting of: continental type cheese, cheddar, mascarpone, pasta filata, mozzarella, pizza cheese, feta, soft cheese, brie, camembert, fresh cheese, cottage cheese and gouda.

In an embodiment of the present invention, the milk to be coagulated has a pH in the range of 6.0 to 7.0, such as in the range 6.3 to 7.0, or presently preferred in the range of 6.3 to 6.9, such as in the range of 6.4 to 6.8 or 6.5 to 6.7.

The bovine milk is milk from an animal species selected from the group consisting of: cow, buffalo, sheep or goat; or the milk is a composition which comprises milk from at least of one said animal species. It is presently preferred that the bovine milk is cow's milk; or the milk is a composition which comprises cow's milk.

The time for curd formation depends on e.g. the type of cheese, the temperature of the milk, the pH, and the concentration of the chymosin enzyme. The skilled person has the ability to modify the time needed, and therefore such modifications are a part of the present invention. Normally, the time for curd formation is within the range of 10 to 60 minutes, such as in the range of 20 to 40 minutes, and normally the temperature is within the range of 25 to 40 degrees C when the coagulant is mixed with the milk. Thus, the milk may be tempered (before or after contacting with chymosin) to a temperature in the range 20 to 50 degrees C.

The chymosin enzyme (or the DNA sequence encoding it) may be obtained from several sources. In an embodiment of the present invention the animal of the suborder *Tylopoda* is an animal belongs to the family *Camelidae,* and in a further embodiment the animal belongs to a species selected from the group consisting of *Camelus dromedarius, Camelus bactrianus* and *Lama glama.* In an other embodiment, the chymosin has a sequence identity of at least 90% (preferable at least 95%, more preferable at least 98% or even more preferable at least 99%) to the amino acid sequence 59-381 of any of the sequences: SEQ ID No: 1, SEQ ID No: 2 or SEQ ID No: 3.

| | |
|---|---|
| C._bactrianus | 1                                                  50 MRCLVVLLAA LALSQASGIT RIPLHKGKTL RKALKERGLL EDFLQRQQYA MRCLVVLLAA LALSQASGIT RIPLHKGKTL RKALKERGLL EDFLQRQQYA MRCLVVLLAA LALSQASGIT RIPLYKGKTL RKALKEHGLL EDFLQRQQYA |
| Camelus_ dromedarius | |
| Lama glama | |
| | 51 100 |

| | |
|---|---|
| C._bactrianus | VSSKYSSLGK VAREPLTSYL DSQYFGKIYI GTPPQEFTVV FDTGSSDLWV VSSKYSSLGK VAREPLTSYL DSQYFGKIYI GTPPQEFTVV FDTGSSDLWV VSSKYSSLGK VAREPLTSYL DSQYFGKIYI GTPPQEFTVV FDTGSSDLWV |
| Camelus_dromedarius | |
| Lama | |
| C._bactrianus | 101                                                150 PSIYCKSNAC KNHHRFDPRK SSTFRNLGKP LSIHYGTGSI EGFLGYDTVT PSIYCKSNVC KNHHRFDPRK SSTFRNLGKP LSIHYGTGSM EGFLGYDTVT PSIYCKSNVC KNHHRFDPRK SSTFRNLGKP LSIHYGTGSM EGFLGYDTVT |
| Camelus_dromedarius | |
| Lama | |
| C._bactrianus | 151                                                200 VSNIVDPNQT VGLSTEQPGE VFTYSEFDGI LGLAYPSLAS EYSVPVFDNM VSNIVDPNQT VGLSTEQPGE VFTYSEFDGI LGLAYPSLAS EYSVPVFDNM VSNIVDPNQT VGLSTEQPGE VFTYSEFDGN LGLAYPSLAS EYSVPVFDNM |
| Camelus_dromedarius | |
| Lama | |
| C._bactrianus | 201                                                    250 MDRHLVARDL FSVYMDRNGQ GSMLTLGATD PSYYTGSLHW VPVTVQQYWQ MDRHLVARDL FSVYMDRNGQ GSMLTLGAID PSYYTGSLHW VPVTLQQYWQ MDRHLVAQDL FSVYMDRNGQ GSMLTLGAID SSYYTGSLHW VPVTVQQYWQ |
| Camelus_dromedarius | |
| Lama | |
| C._bactrianus | 251                                                    300 VTVDSVTING VAVACVGGCQ AILDTGTSVL FGPSSDILKI QMAIGATENR FTVDSVTING VAVACVGGCQ AILDTGTSVL FGPSSDILKI QMAIGATENR VTVDSVTING VAVACVGGCQ AILDTGTSVL FGPSSDILKI QKAIGATENR |
| Camelus_dromedarius | |
| Lama | |
| C._bactrianus | 301                                              350 YGEFDVNCGS LRSMPTVVFE INGRDFPLAP SAYTSKDQGF CTSGFQGDNN YGEFDVNCGN LRSMPTVVFE INGRDYPLSP SAYTSKDQGF CTSGFQGDNN YGEFDVNCGN LRSMPTVVFE INGRDYPLSP SAYTSKDQGF CTSGFQGDNN |
| Camelus_dromedarius | |
| Lama | |
| C._bactrianus | 351                                    381 SELWILGDVF IREYYSVFDR ANNRVGLAKA I SELWILGDVF IREYYSVFDR ANNRVGLAKA I SELWILGDVF IREYYSVFDR ANNRVGLAKA I |
| Camelus_dromedarius | |
| Lama | |

In a further embodiment, the chymosin has a sequence identity of at least 90% (preferable at least 95%, more preferable at least 98% or even more preferable at least 99%) to the *Lama guanicoe* chymosin amino acid sequence SEQ ID No: 4:

GKVAREPLTS YLDSQYFGKI YIGTPPQEFT VVFDTGSSDL WVPSIYCKSN ACXXXXXXXX XXXXXXXXXX

XXXXXXXXXX XXXXXXXXXX XXVSNIVDPN QTVGLSTEQP GEVFTYSEFD GILGLAYPSL ASEYSVPVFD

NMMDRHLVAQ DLFSVYMDXX XXXXXXXXXX XXXXXXXXXX XXXXXXXXXX XXXXXXXVTI NGVAVACVGG

CQAILDTGTS VLFGPSSDIL KIQMAIGATE NRYGEFDVNC GNLRSMPTVV FEINGRDFPL APSAYTSKDQ

GFCTSGFQSE NHSQKWILGD VFIREYYSVF DRANNLVGLA KAI

In a presently preferred embodiment, the chymosin has a sequence identity of at least 90% (preferable at least 95%, more preferable at least 98% or most preferable at least 99%) to the amino acid (aa) sequence 59-381 of the depicted SEQ ID No: 1: 
1 MRCLVVLLAA LALSQASGIT RIPLHKGKTL RKALKERGLL EDFLQRQQYA VSSKYSSLGK

61 VAREPLTSYL DSQYFGKIYI GTPPQEFTVV FDTGSSDLWV PSIYCKSNVC KNHHRFDPRK

121 SSTFRNLGKP LSIHYGTGSM EGFLGYDTVT VSNIVDPNQT VGLSTEQPGE VFTYSEFDGI

181 LGLAYPSLAS EYSVPVFDNM MDRHLVARDL FSVYMDRNGQ GSMLTLGAID PSYYTGSLHW

241 VPVTLQQYWQ FTVDSVTING VAVACVGGCQ AILDTGTSVL FGPSSDILKI QMAIGATENR

301 YGEFDVNCGN LRSMPTVVFE INGRDYPLSP SAYTSKDQGF CTSGFQGDNN SELWILGDVF

361 IREYYSVFDR ANNRVGLAKA I
 or the chymosin contains an amino acid sequence which has a sequence identity of at least 95% (preferable at least 96%, more preferable at least 98% or most preferable at least 99%) to any 50 aa length fragments of the sequence 59-381 of SEQ ID No: 1.

By comparing the amino acid sequences common for *Tylopoda* species - but absent in bovine prochymosin (see the above comparative sequence listing) - it becomes clear that sequence differences between bovine and *Tylopoda* prochymosin are spread all over the molecule. However, three areas of special interest can be defined. All amino acid numbers refer to the numbering in the above sequence listing;
- aa 57-68, with 6 *Tylopoda* specific amino acids. The differences between *Tylopoda* and bovine chymosin in this area result in a remarkable change in charge. These comprise the first amino acids of the mature chymosin molecule
- aa 160-161. Two very exposed amino acid residues at the backbone of the molecule.
- aa 301-329. Most differences between *Tylopoda* and bovine prochymosins are located at the C-terminal part of the molecule. The 301-329 area is located at the entrance of the catalytic cleft and is likely to be responsible for interaction with the casein substrate of the molecule.
Based on the evaluation described below it is most likely that the sequence variation at the amino acid positions 301-329 is responsible for some of the functional differences between bovine and *Tylopoda* chymosins.

Most differences are found in all four *Tylopoda* species analyzed. There are only two cases in which both *Camelus* sequences differ from the two *Lama* sequences (in both cases the *Camelus* chymosins have an 'R' while the *Lama* chymosins have H in one case and Q in the other case). Based on this comparison it is unlikely that major differences will be found in the functional properties of different *Tylopoda* chymosin molecules.

It is contemplated a part of the *Tylopoda* chymosins that gives the superior properties is the sequence starting at aa 301, and ending at aa 329. Therefore, a preferred embodiment of the present invention relates to method, wherein the chymosin contains an amino acid sequence selected from the group consisting of: SEQ ID No: 5 (YGEFDVNCGS LRSMPTVVFE INGRDFPLAP), SEQ ID No: 6 (YGEFDVNCGN LRSMPTVVFE INGRDYPLSP), and SEQ ID No: 7 (YGEFDVNCGN LRSMPTVVFE INGRDYPLSP). The amino acid sequence of the rest of the enzyme (aa 59 to 300 and 330-381) to may be substantially identical to the same parts of any chymosin amino acid sequence.

In the presently most preferred embodiment, the chymosin has the amino acid sequence 59-381 of SEQ ID No: 1.

The enzyme may be prepared by any method known to the skilled person, e.g. by extraction from abomasum tissue or by recombinant DNA techniques, wherein the chymosin is produced using a bacteria such as *E. coli,* a yeast; or a fungus (including a filamentous fungus) as host organism. The fungus may be an *Aspergillus* species, such as *Aspergillus niger.*

It is contemplated that the chymosin enzyme of the invention may be used as the only coagulant, or a further kappa-casein cleaving enzyme may be contacted with the milk, such as a bovine chymosin, a peptidase or a microbial coagulant. It is anticipated that the further enzyme may used in an amount of up to 50% (such as up to 5%, up to 10%, up to 20%, up to 30% or up to 40%), measured in IMCU, of the *Tylopoda* chymosin. However, it is presently not desired that the milk is contacted with a camel pepsin (EC 3.4.23.1, especially the pepsin obtainable from camel calf abomasum) in an amount exceeding 30% (such as exceeding 20%, exceeding 10% or exceeding 5%), measured in IMCU, of the chymosin. In a specific embodiment, the milk is not contacted with camel pepsin, especially the pepsin obtainable from camel calf abomasum.

Prior to the present invention, the only *Tylopoda* chymosin enzyme that has been contacted with bovine milk seems to be camel chymosin, but it has apparently not been used for cheese production. Thus, in a further aspect the present invention relates to a method for manufacturing cheese comprising contacting bovine milk with a chymosin enzyme having an amino acid sequence identical or substantially identical to the amino acid sequence of chymosin (EC 3.4.23.4) from an animal of the suborder *Tylopoda.* In a presently preferred embodiment, it is preferred that the enzyme is not identical to SEQ ID No 1, amino acids 59-381, or is not a naturally occurring camel chymosin (purified from the abomasum of the animal).

It a still further aspect, the invention relates to a chymosin enzyme having an amino acid sequence identical or substantially identical to the amino acid sequence of chymosin (EC 3.4.23.4) from an animal of the suborder *Tylopoda,* provided that said chymosin is not identical to SEQ ID No 1, amino acids 59-381. The enzyme may be in glucosylated or unglycosylated form. It is contemplated that when the enzyme is produced in an *Aspergillus* host cell, it will have an other glycosylation pattern than the enzyme that may be purified from the abomasum of the animal.

Further, the invention relates to a method for manufacturing a dairy product (e.g. cheese or curd), comprising contacting bovine milk with a chymosin enzyme having an amino acid sequence identical or substantially identical to the amino acid sequence of chymosin (EC 3.4.23.4) from an animal of the suborder *Tylopoda,* said chymosin is added in an amount not exceeding 30 IMCU per liter milk and/or said chymosin is added in an amount not exceeding 90% of the amount (measured in IMCU) of bovine chymosin B that would have been necessary for obtaining a curd with the same strength (firmness) in the same time and at the same temperature using the same milk; to a method for manufacturing a dairy product (eg cheese or curd) comprising obtaining a curd (such as with a firmness of 20mm +/- 5mm measured on Formagraph equipment) by contacting bovine milk with *Tylopoda* chymosin at a concentration (amount) below 950 IMCU multiplied by volume of milk in liter and divided by desired time for coagulation (cutting time, ie time from addition of chymosin to cutting) in minutes (i.e. calculated as 950 x L / minutes); and to a method for improving taste and/or texture of a dairy product (e.g. cheese or yoghurt), comprising contacting bovine milk with a chymosin enzyme having an amino acid sequence identical or substantially identical to the amino acid sequence of chymosin (EC 3.4.23.4) from an animal of the suborder *Tylopoda.*

Presently preferred embodiments of the methods of the invention are:
- The dairy product may be cheese, eg a continental type cheese, e.g. emmenthaler, danbo, gouda, havarti, tilsit; a pasta filata type cheese (eg mozzarella, pizza cheese); cheddar type cheese, mascarpone, feta, soft cheese, brie, camembert, fresh cheese, cottage cheese.
- The milk may have a pH in the range of 6.0 to 7.0, more preferred in the range 6.2 to 6.8, or most preferred in the range 6.4 to 6.6, such as in a range selected from: 6.3 to 7.0, 6.3 to 6.7, 6.5 to 6.6, 6.3 to 6.9, 6.4 to 6.8, 6.2 to 6.5, and 6.5 to 6.7.
- The milk may have a temperature within the range 25-37 degrees C (such as in the range 29-35 degrees C or in the range 31-33 degrees C).
- The milk may be from an animal species selected from the group consisting of: cow, buffalo, sheep and goat; or the milk is a composition which comprises milk from at least of one animal species selected from the group consisting of: cow, buffalo, sheep and goat. It is presently preferred that the milk is cow's milk; or the milk is a composition which comprises cow's milk.
- The chymosin enzyme may used in an amount below 6.5 mg per 100 liter of milk, and/or used in the ratio from 5 to 25 IMCU per liter of milk, e.g. 10-20 IMCU per liter.
- The time for curd formation may in the range of 10 to 60 minutes, such as in the range of 20 to 40 minutes.
- The animal of the suborder *Tylopoda* may be an animal belonging to the family *Camelidae,* such as a species selected from the group consisting of *Camelus dromedarius, Camelus bactrianus* and *Lama glama.*
- The chymosin may have a sequence identity of at least 90% (preferable at least 95%, more preferable at least 98% or even more preferable at least 99%) to the amino acid sequence 59-381 of any of the sequences: SEQ ID No: 1, SEQ ID No: 2, or SEQ ID No: 3, and/or a sequence identity of at least 90% (preferable at least 95%, more preferable at least 98% or most preferable at least 99%) to the aa (amino acid) sequence 59-381 of SEQ ID No: 1.
- The chymosin may contain an amino acid sequence which has a sequence identity of at least 95% (preferable at least 96%, more preferable at least 98% or most preferable at least 99%) to any 50 aa length fragments of the sequence 59-381 of SEQ ID No: 1.
- The chymosin contains an amino acid sequence selected from the group consisting of: SEQ ID No: 5, SEQ ID No: 6, and SEQ ID No: 7.
- The chymosin may have the amino acid sequence 59-381 of SEQ ID No: 1.
- The chymosin may be produced using a bacteria, a yeast; or a fungus (including a filamentous fungus) as host organism.
- The fungus may be an *Aspergillus* species, such as *Aspergillus niger.*
- A further enzyme, e.g. a kappa-casein cleaving enzyme, may be contacted with the milk, such as a bovine chymosin, a peptidase, a protease, or a microbial or animal coagulant. The further enzyme amy be used in an amount of up to 50% (such as up to 5%, up to 10%, up to 20%, up to 30% or up to 40%), measured in IMCU, of the *Tylopoda* chymosin.
- The milk may be not contacted with a camel pepsin (EC 3.4.23.1, especially the pepsin obtainable from camel calf abomasum) in an amount exceeding 30% (such as exceeding 20%, exceeding 10% or exceeding 5%), measured in IMCU, of the chymosin.
- The milk may not be contacted with camel pepsin, especially the pepsin obtainable from camel calf abomasum.
- The milk may be tempered (before or after contacting with chymosin) to a temperature in the range 20 to 50 degrees C, such as in the range 25 to 40 degrees C.

In a still further aspect, the invention relates to a method for manufacturing cheese comprising contacting bovine milk with a chymosin enzyme having an amino acid sequence identical or substantially identical to the amino acid sequence of chymosin (EC 3.4.23.4) from an animal of the suborder *Tylopoda,* or to one or more of the following sequences: SEQ ID No: 1; SEQ ID No: 2; SEQ ID No: 3; and SEQ ID No: 4. In the method, it may be preferred that said chymosin is not identical to SEQ ID No 1, amino acids 59-381.

Also, the invention relates to a chymosin enzyme having an amino acid sequence identical to or having a sequence identity of at least 90% to the amino acid sequence of chymosin (EC 3.4.23.4) from an animal of the suborder *Tylopoda,* or to one or more of the following sequences: SEQ ID No: 1; SEQ ID No: 2; SEQ ID No: 3; and SEQ ID No: 4. It may be preferred that said chymosin enzyme is not identical to the amino acid (aa) sequence 59-381 of SEQ ID No: 1.

An aspect of the present invention is a dairy product (such as cheese or yoghurt) obtainable by any method according to the invention, such as a continental type cheese or a cheddar cheese.

In a last aspect, the present invention relates to a DNA sequence encoding a *Tylopoda* chymosin enzyme, i.e. a sequence that is substantially identical (eg having a sequence identity of at least 90%, preferably at least 92%, more preferably at least 94%, even more preferably at least 96%, even more preferably at least 98%, and even more preferably 99% or 100%) to one of the following sequences (the alignment of two sequences and the calculation of nucleotide identity may be done as described in US patent 6162628), and/or is able to hybridize to one of the complementary sequences thereto under stringent conditions:
SEQ ID No: 8 *Camelus dromedarius* 
gacggtgactgacacgtggcggagtgggatcaccaggatccctctgcacaaaggcaagactctgagaaaagcgctgaaggagcgt
gggctcctggaggactttctgcagagacaacagtatgccgtcagcagcaagtactccagcttggggaaggtggccagggaacccctg
accagctacctggatagtcagtactttgggaagatctacatcggaccccaccccaggagttcaccgtggtgtttgacactggctcctct
gacctgtgggtgccctctatctactgcaagagcaatgtctgcaaaaaccaccaccgctttgacccgagaaagtcgtccaccttccggaa
cctgggcaagcccctgtccatccattacggcacgggcagcatggagggctttctgggctacgacaccgtcaccgtctccaacattgtgg
accccaaccagactgtgggcctgagcaccgagcaacctggcgaggtcttcacctactccgagtttgacggatcctggggctggccta
cccctcgcttgcctccgagtactcggtgcccgtgtttgacaatatgatggacagacacctggtggcccgagacctgttctcggtttacatg
gacaggaatggccaggggagcatgcttacactgggggccattgacccgtcctactacaccggctccctgcactgggtgcccgtgacct
tgcagcagtactggcagttcaccgtggacagtgtcaccatcaacggggtggcagtggcctgtgttggtggctgtcaggccatcctgga
cacgggtacctccgtgctgttcgggcccagcagcgacatcctcaaaattcagatggctattggagccacagagaaccgatatggtgag
tttgacgtcaactgtgggaacctgaggagcatgcccaccgtggtcttcgagatcaatggcagagactacccactgtcccctccgccta
cacaagcaaggaccagggcttctgcaccagtggctttcaaggtgacaacaattccgagctgtggatcctgggggatgtcttcatccgg
gagtattacagtgtctttgacagggccaacaatcgcgtggggctggccaaggccatctgatcctctagagtcg
SEQ ID No: 9 *Camelus bactrianus* preprochymosin 
atgcggtgcctcgtggtgctacttgcagccctcgctctcccaggccagtgggatcaccaggatccctctgcacaaaggcaagactct
gagaaaagcgctgaaggagcgtggctcctggaggactttctgcagagacaacagtatgccgtcagcagcaagtactccagcttgg
ggaaggtggccagggaacccctgaccagctacctggatagtcagtactttgggaagatctacatcgggaccccaccccaggagttca
ccgtggtgtttgacactggctcctctgacctgtgggtgccctctatctactgcaagagcaatgcctgcaaaaaccaccaccgctttgacc
cgagaaagtcgtccaccttccggaacctgggcaagcccctgtccatccattacggcacgggcagcattgagggctttctgggctacga
caccgtcaccgtctccaacattgtggaccccaaccagactgtgggcctgagcaccgagcaacctggcgaggtcttcacctactccgagt
ttgacgggatcctggggctggcctacccctcgcttgcctccgagtactcggtgcccgtgtttgacaatatgatggacagacacctggtg
 
gcccgagacctgttctcggtttacatggacaggaatggccaggggagcatgcttacactgggggccactgacccctcctactacaccg
gctccctgcactgggtgcccgtgaccgtgcagcagtactggcaggtcaccgtggacagtgtcaccatcaacggggtggcagtggcct
gtgttggtggctgtcaggccatcctggacacgggtacctccgtgctgttcgggcccagcagcgacatcctcaaaattcagatggctatt
ggagccacagagaaccgatatggtgagtttgacgtcaactgtgggagcctgaggagcatgcccaccgtggtcttcgagatcaatggc
agagacttcccactggcccccctccgcctacacaagcaaggaccagggcttctgcaccagtggctttcaaggtgacaacaattccgagc
tgtggatcctgggggatgtcttcatccgggagtattacagtgtctttgacagggccaacaatcgcgtggggctggccaaggccatctga
tcctctagagtcg
SEQ ID No: 10 *Lama glama* chymosin 
acgtcgaccgcgacggtgactgacacgtggcgtgccgagatcactcgcatccccctctacaagggcaagcctctgcgtaaggctctca
aggagcgcggtctgctcgaggatttcctgcagaagcagcagtacggcgtcagctctgagtacagcggtttcggcgaggtggccagcg
tgcctctcactaactacctggacagccagtacttcggtaagatctaccttggcactccccctcaggagttcaccgttctgttcgatactggt
tccagcgacttctgggttccctccatctactgtaagagcaacgcttgcaagaaccaccagcgcttcgatcctcgcaagtccagcaccttc
cagaaccttggcaagcccctttccatccgctacggtactggcagcatgcagggtatccttggctacgacaccgttaccgtgtccaacatc
gtcgatattcagcagaccgtgggtctgagcacccaggagcctggcgatgtcttcacttacgccgagttcgatggtatcctcggcatggc
ttacccctccctggcctctgagtactctgtccctgtgttcgacaacatgatggaccgccgcctcgtcgctcaggatctgttcagcgtgtaca
tggaccgtagcggtcaggggtccatgcttactctgggcgccatcgatccctcttactacaccggttccctccactgggttcctgtgaccct
ccagaagtactggcagttcaccgtggacagcgtcactatctccggcgcggttgtggcttgcgagggtggctgtcaggccatccttgata
ctggtaccagcaagctcgtcggcccctccagcgacatcctgaacatccagcaggctatcggtgccacccagaaccagtacggcgagtt
cgatatcgactgcgatagcctttcttccatgcctactgtggttttcgagatcaacggtaagatgtacccccttactccttatgcttacacttc
ccaggaggagggcttctgtacctctggtttccagggtgagaaccacagccaccagtggatccttggcgatgtcttcatccgcgagtact
actccgtcttcgaccgtgccaacaacctggtgggtctcgctaaggccatctgatcctctagagtc
SEQ ID No: 11 *Lama guanicoe* chymosin, including part of the prosequence

nncttgatccctctgtacaaaggcaagactctgagaaaagcgttgaaggagcatggctcctggaggactttctgcagagacaacag
tatgccgtcagcagcaagtactccagcttggggaaggtggccagggaacccctgaccagctacctggatagtcagtactttgggaag
atctacatcgggaccccaccccaggagttcaccgtggtgtttgacactggctcctccgacctgtgggtgccctctatctactgcaagagc
aatgcctgcaannnnnnnnnnnnnnnnnnnnnnnnnnnnnnnnnnnnnnnnnnnnnnnnnnnnnnnnnnnnnnnnnnnnnnnnnnnnn
nnnnnnnnnnnnnnnnnnnnnnnnnnnnnnnnnnnnnnnnnnnnnnnnnnnnnnnnnnnnnnnnnnnnnnngtctccaacattgtgga
ccccaaccagactgtgggcctgagcaccgagcaacctggcgaggtcttcacctactccgaatttgacggatcctggggctggcctac
ccctcgcttgcctccgagtactcggtgcccgtgtttgacaatatgatggacagacacctggtggcccaagacctgttctcggtttacatg
gacagnnnnnnnnnnnnnnnnnnnnnnnnnnnnnnnnnnnnnnnnnnnnnnnnnnnnnnnnnnnnnnnnnnnnnnnnnnnnnnnnnnn
nnnnnnnnnnnnnnnnnnnnnnnnnnnnnnnnnnnnnnnnnnnnnnnnnntgtcaccatcaacggggtggcagtggcctgtgttggtg
gctgtcaggccatcctggacacgggtacctccgtgctgttcgggcccagcagcgacatcctcaaaattcagatggctattggagccaca
gagaaccgatatggtgagtttgacgtcaactgtgggaacctgaggagcatgcccaccgtggtcttcgagatcaatggcagagacttcc
cactggcccccctccgcctacacaagcaaggaccagggcttctgcaccagtggcttccagagtgaaaatcattcccagaaatggatcct
gggggatgttttcatccgagagtattacagcgtctttgacagggccaacaacctcgtggggctggccaaggccatctga

*Lama guanicoe* chymosin amino acid sequence, including part of the prosequence
---Exon 1 ----
   liplykgktlrkalkehglledflqrqqyavsskysslgkvarepltsyldsqyfgkiyigtppqeftvvfdtgssdlwvpsiycksnac
------Exon 4---------------
   vsnivdpnqtvglsteqpgevftysefdgilglaypslaseysvpvfdnmmdrhlvaqdlfsvymdx
----Exon 6 ---------------

vtingvavacvggcqaildtgtsvlfgpssdilkiqmaigatenrygefdvncgnlrsmptvvfeingrdfplapsaytskdqgfctsgf
qsenhsqkwilgdvfireyysvfdrannlvglakai*

### DEFINITIONS

In the present context, the term "chymosin" refers to an enzyme (EC 3.4.23.4) able to clot milk by cleaving the scissile bond in kappa-casein, and it is preferred that the enzyme combines a strong clotting activity with a low general proteolytic activity.

The chymosin of the present invention preferably has at least 85%, more preferably at least 90%, even most preferably at least 95%, even most preferably at least 100%, and even most preferably 110% of the kappa-casein cleaving activity of the polypeptide consisting of the amino acid sequence 59-381 of SEQ ID No: 1. SEQ ID No: 1 is the amino acid sequence of *Camelus dromedarius* prochymosin. The N-terminal amino acid of the mature chymosin enzyme is Gly(59). Thus, the mature chymosin has the amino acid sequence: GK VAREPLTSYL DSQYFGKIYI GTPPQEFTVV FDTGSSDLWV PSIYCKSNVC KNHHRFDPRK SSTFRNLGKP LSIHYGTGSM EGFLGYDTVT VSNIVDPNQT VGLSTEQPGE VFTYSEFDGI LGLAYPSLAS EYSVPVFDNM MDRHLVARDL FSVYMDRNGQ GSMLTLGAID PSYYTGSLHW VPVTLQQYWQ FTVDSVTING VAVACVGGCQ AILDTGTSVL FGPSSDILKI QMAIGATENR YGEFDVNCGN LRSMPTVVFE INGRDYPLSP SAYTSKDQGF CTSGFQGDNN SELWILGDVF IREYYSVFDR ANNRVGLAKA I

The chymosin used in the process of the invention has an amino acid sequence identical or substantially identical to the amino acid sequence of chymosin (EC 3.4.23.4) from an animal of the suborder *Tylopoda,* which includes *Camelidae* species such as *Camelus dromedarius* (arabian camel) and *Camelus bactrianus* (bactrian camel), *Vicugna* species such as *Vicugna vicugna* (vicugna), and *Lama* species such as *Lama glama* (llama), *Lama guanicoe* (guanaco) and *Lama paco* (alpaca). The term "substantially identical" refers to that the chymosin of the present invention has an amino acid sequence which has a sequence identity of at least 90%, preferably at least 92%, more preferably at least 94%, even more preferably at least 96%, even more preferably at least 98%, and even more preferably 99% or 100% to the amino acid sequence of the mature chymosin (or an amino acid sequence that in situ can be converted to the mature chymosin) from an animal of the *Tylopoda* suborder, i.e. naturally present in the stomach of said animal. In a preferred embodiment, the chymosin of the present invention has an amino acid sequence which has a sequence identity of at least 90%, preferably at least 92%, more preferably at least 94%, even most preferably at least 96%, even most preferably at least 98%, and even most preferably 99% to the amino acid sequence 59-381 of SEQ ID No: 1. The relatedness between two amino acid sequences is described by the parameter "identity". For purposes of the present invention, the degree of identity between two amino acid sequences is determined by the Clustal method (Higgins, 1989, CABIOS 5: 151-153) using the LASERGENE™ MEGALIGN™ software (DNASTAR, Inc., Madison, WI) with an identity table and the following multiple alignment parameters: Gap penalty of 10 and gap length penalty of 10. Pairwise alignment parameters are Ktuple=1, gap penalty=3, windows=5, and diagonals=5.

Chymosin of the present invention is preferable obtained by so called recombinant DNA techniques, i.e. from host cells that have been transformed by a DNA construct comprising the DNA sequence for the enzyme (or a proform thereof) to produce chymosin (or proforms thereof). Typical host cells can be of microbial origin, such as yeast, fungi (in particular Aspergillus niger), bacteria etc without exclusion of mammalian cells. Recombinant camel chymosin may be obtained as disclosed in WO 02/36752, and it is within the basic knowledge of a skilled person to produce chymosins that are substantially identical to the camel chymosin by modifying the DNA sequence encoding camel chymosin and inserting the sequence into an appropriate vector, e.g. the vector disclosed in WO 02/36752.

The chymosin of the present invention is preferably substantially pure, and in the method of the invention, milk is contacting with a substantially pure chymosin. The term "substantially pure chymosin" denotes herein a chymosin preparation which contains at most 10%, preferably at most 8%, more preferably at most 6%, more preferably at most 5%, more preferably at most 4%, more preferably at most 3%, even more preferably at most 2%, most preferably at most 1%, and even most preferably at most 0.5% by weight of other polypeptide material with which it is natively associated. It is, therefore, preferred that the substantially pure chymosin is at least 92% pure, preferably at least 94% pure, more preferably at least 95% pure, more preferably at least 96% pure, more preferably at least 96% pure, more preferably at least 97% pure, more preferably at least 98% pure, even more preferably at least 99%, most preferably at least 99.5% pure, and even most preferably 100% pure by weight of the total polypeptide material present in the preparation. This can be accomplished, for example, by preparing the chymosin by means of well-known recombinant methods or by classical purification methods.

By the term "bovine milk" is understood a composition comprising milk from an animal species belonging to the subfamily *Bovinae* (which includes the domestic cow (*Bos taurus*) and buffalo). The composition may consist entirely of milk from an animal species belonging to the subfamily *Bovinae* (e.g. cow milk or buffalo milk), or it may as a major part (eg. more than 70%, more than 80% or even more than 90% (v/v)) comprise milk from an animal species belonging the subfamily *Bovinae.* Specifically, the composition may besides milk from an animal species belonging to the subfamily *Bovinae* comprise milk from an animal species belonging to the subfamily *Caprinae* (which includes goat and sheep). Also, the bovine milk may be a dairy product made from the above defined milk composition (such as fermented by addition of a lactic acid bacterium), or whey. Optionally the milk is acidified, e.g. by addition of an acid (such as citric, acetic or lactic acid) or by addition of an acid producing microorganism. The milk may be raw or processed, e.g. by filtering, sterilizing, pasteurizing, homogenizing etc, or it may be reconstituted dried milk. Important examples of "bovine milk" according to the present invention is pasteurized cow's milk which is optionally fermented with a lactic acid bacterium culture. It is understood that the milk may be acidified, mixed or processed before, during and/or after the contacting with the chymosin enzyme.

By the term "yoghurt" is understood fermented milk, ie. a milk based product which is fermented by addition of one or more lactic acid bacterial strains.

The term "cheese" refers to a product prepared by contacting optionally acidified milk (eg by means of a lactic acid bacterial culture) with a coagulant, and draining the resultant curd. Cheeses and their preparation are described in eg Cheese and Fermented Milk Foods, by Frank V. Kosikowski. The term "cheese of the continental type" should be understood as cheeses of the types, such as Gouda, Danbo, Edam, St. Paulin, Raclette, Fontal etc and/or cheeses made by a process which may include heating the curd to a temperature that does not exceed 45 degrees C. The term "cheese of the cheddar type" should be understood as cheeses of the types such as Cheddar, Territorials, American Cheddar, Monterey Jack and Colby, and/or cheeses made by a process which may include heating the curd to a temperature that does not exceed 45 degrees C.

By the term "IMCU" is understood International milk clotting units. One IMCU equals about 0.126 nmol of bovine chymosin B (eg CHY-MAX). The strength of a milk clotting enzyme (such as a chymosin enzyme of the present invention) is determined as the milk clotting activity (IMCU per ml or pr g). Following the addition of diluted coagulant to a standard milk substrate, the milk will flocculate. The milk clotting time is the time period from addition of the coagulant until formation of visible flocks or flakes in the milk substrate. The strength of a coagulant sample is found by comparing the milk clotting time for the sample to that of a reference standard, a normal. This is expressed in IDF standard 157A:1997 which gives the IMCU definition: The total milk-clotting activity of the first batch of calf chymosin reference standard powder has once and for all been set at 1000 International Milk-Clotting Units per gram (IMCU/g). Further preparations of reference standards will be set relative to the previous reference.
IMCU principle: Determination of the time needed for visible flocculation of renneted standard milk substrate with 0.05% calcium chloride, pH 6.5. IMCU/ml of a sample is determined by comparison of the clotting time to that of a reference standard having known milk-clotting activity and having the same enzyme composition as the sample.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

### LEGENDS TO FIGURES

Figure 1. Formagraph curd formation of calf and camel chymosin in non-homogenized whole milk adjusted to pH 6.5 and 32 degrees C. Dosage of both coagulants was 3250 IMCU/100L.
Figure 1a is a variant of figure 1 wherein the values for typical cutting firmness, time for clotting, and typical time of cutting (for both camel chymosin and bovine calf chymosin B) are marked.
Figure 2. Dosage difference (in IMCU) between CHY-MAX M (camel chymosin) and CHY-MAX (calf chymosin) in organic whole milk (pH 6.6 and 32°C). Time to cutting measured as 20 mm firmness on Formagraph equipment.

### EXAMPLES

### Example 1:

### Curd formation

Curd formation of calf and camel chymosin in non-homogenized whole milk adjusted to pH 6.5 and 32 degrees C was measured using a Formagraph (Foss Electric, Denmark). Dosage of both coagulants was 3250 IMCU/100L.

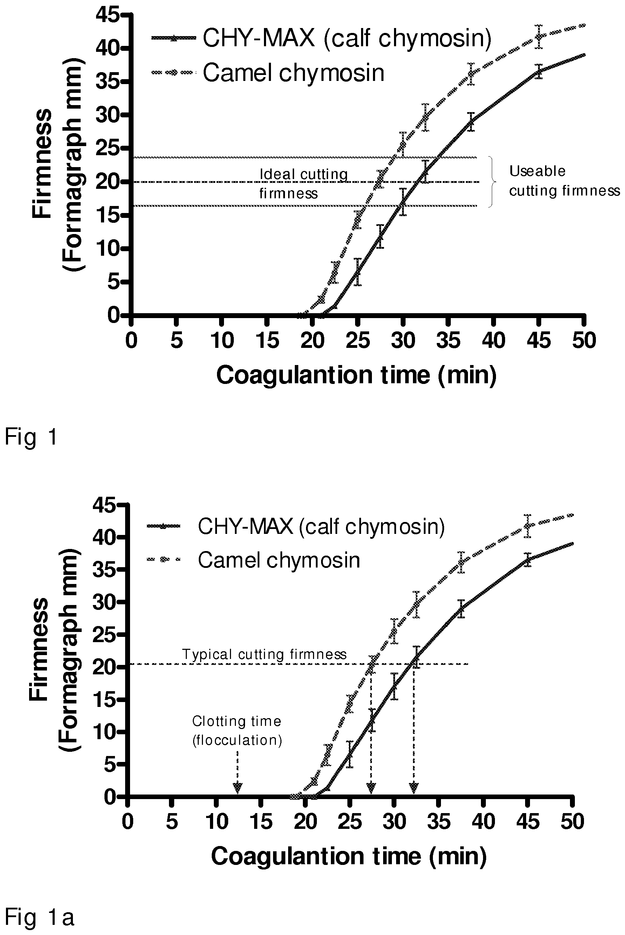

Figure 1 demonstrates clearly a faster build-up of the curd firmness with camel chymosin compared to calf chymosin using the same amount of IMCU, as the ideal cutting firmness is obtained approx. 4 minutes earlier.

### Dosage is dependant of pH

Dosage difference (in IMCU) between CHY-MAX M (camel chymosin) and CHY-MAX® (calf chymosin) in organic whole milk (pH 6.6 and 32°C) is measured using a Formagraph. The cutting time is measured as the time necessary to reach 20 mm firmness on the Formagraph equipment.

The dosage difference is dependant on coagulation/setting pH (interval pH 6.3 to 6.7). Highest dosage difference is obtained at pH 6.7 (approx. 20-30%) and lowest at pH 6.3 (approx. 5-10%). Typical setting pH for Gouda, Cheddar and Pizza cheese is pH 6.4 to 6.6. Figure 2 demonstrates that approx. 27% less IMCU are needed using CHY-MAX M compared to CHY-MAX® in order to reach the same curd firmness within same time.

### Example 2:

### Cheddar trials with camel chymosin

The cheeses were made using a standard make procedure: Cheddar cheeses were made from pasteurized (72 degrees C x 15 s), standardized cows' milk and acidified with Chr. Hansen's starter R604. Control cheeses were coagulated using Chr. Hansen's recombinant (calf) chymosin (CHY-MAX®). The cheeses of the present invention were coagulated with camel chymosin (*Camelus dromedarius* chymosin according to the invention) at an amount 30% less, measured in IMCU, than the control cheeses. Cheeses were ripened at 8C for about 6 months. Thus, the only difference between the cheeses of the present invention and the control cheeses is the coagulant used.

### Sensory profile

The replicates of each cheese treatment (camel versus calf chymosin) were generally consistent. Some consistent differences in flavor and texture were noted between cheeses made with camel chymosin and cheeses made with calf chymosin. Although the overall flavor profiles of the two cheese types were generally similar, cheeses made with calf chymosin were characterized by higher intensities of sulfur and brothy flavors and bitter taste compared to cheeses made with camel chymosin. Texture differences between the two cheese types were also evident. Cheeses made with calf chymosin had more degree of breakdown, smoothness of mass and smoothness of mouth coating and were more cohesive and adhesive than cheeses made with camel chymosin. The flavor and texture differences between the two cheese types suggest a difference in degree of proteolysis/protein breakdown.

The experiment in details:
Six previously frozen cheese samples were tested: camel chymosin trial A, camel chymosin trial B, camel chymosin trial C, calf chymosin trial A, calf chymosin trial B, calf chymosin trial C.

### Sensory Evaluation Methods

Texture: A 15-point product-specific descriptive intensity scale for each texture attribute was used; anchored on the left with "not" and on the right with "very". Reference cheeses were provided and evaluation techniques were standardized.
The eight descriptive panelists (females, ages 45-60 y) used for texture analysis are members of the existing contract descriptive analysis panel in the Food Science Department at North Carolina State University. This panel has been highly trained in the Spectrum™ (Meilgaard et al. 1999) method of descriptive analysis for generation of qualitative and quantitative data

Flavor: A 15-point universal intensity scale was used for flavor analysis. Scale ends were anchored on the left with "none" and on the right with "extreme". Reference cheeses were provided and evaluation techniques were standardized.
Twelve panelists (male and female, ages 22-45 y) trained in the Spectrum™ (Meilgaard et al. 1999) method of flavor descriptive analysis for generation of cheese qualitative and quantitative data were used. This panel has been exclusively trained on the sensory analysis of cheese using a previously established cheese flavor language (Drake et al., 2001; 2005)

### Sample Preparation

Texture: Each reference cheese and test cheese was cut into 1.27cm3 cubes and each panelist was provided with 8-10 cubes per sample per replication. Samples were placed in covered 4-ounce portion cups with three-digit codes and stored at 8°C. Samples were tempered to 12°C prior to presentation to the panel. Panelists were provided with distilled, deionized water and unsalted crackers for palate cleansing. Samples were evaluated in triplicate by each panelist.

Flavor: Each test cheese was cut into approximate 2.5 cm3 cubes and each panelist received 1 cube per sample per replication. Samples were placed in covered 2-ounce portion cups with three-digit codes and stored at 8°C. Samples were tempered to 12°C just prior to presentation to the panel. Panelists were provided with distilled, deionized water and unsalted crackers for palate cleansing. Samples were evaluated in triplicate by each panelist.

### Statistical Analysis

Data were evaluated for replicate and treatment effects by analysis of variance with means separation. Principal component analysis was also applied to visualize how all cheeses were differentiated from each other. Analyses were conducted using the SAS Statistical Analysis Software (version 8.2, Cary, NC).

### Results

Some consistent differences in flavor and texture were noted between cheeses made with camel chymosin and cheeses made with calf chymosin. Although the overall flavor profiles of the two cheese types were generally similar, cheeses made with calf chymosin were characterized by higher intensities of sulfur and brothy flavors and bitter taste compared to cheeses made with camel chymosin (p<0.05) (Table 1). Texture differences between the two cheese types were also evident. Cheeses made with calf chymosin had more degree of breakdown, smoothness of mass and smoothness of mouth coating and were more cohesive and adhesive than cheeses made with camel chymosin (p<0.05) (Table 2). The flavor and texture differences between the two cheese types suggest a difference in degree of proteolysis/protein breakdown.

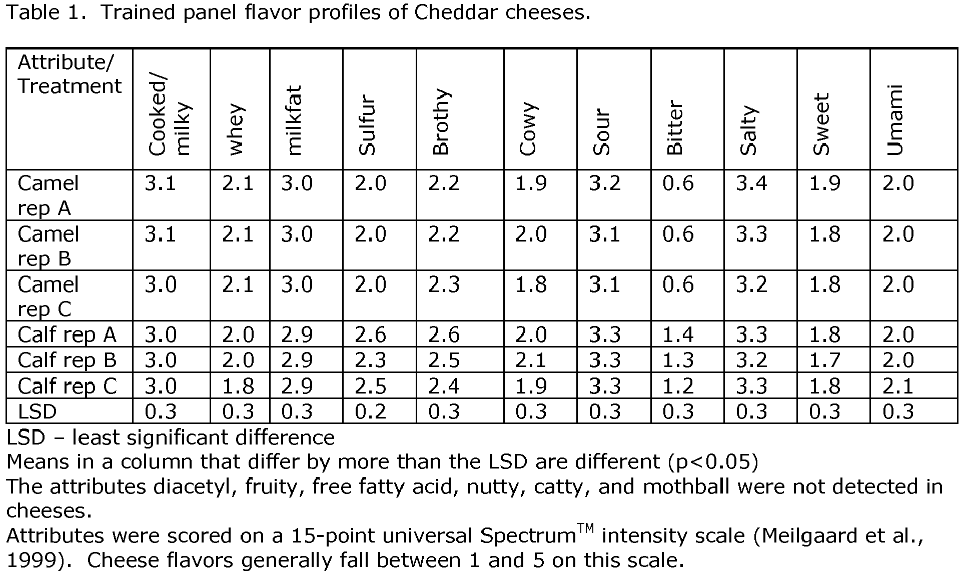

**Table 2. Trained panel texture profiles of Cheddar cheeses.**

| Attribute/ Treatment | HFirm | HSpring | Hrecovery | Firm | Frac | Degree Brkdwn | Coh | Adh | Smth mass | Smth mthct |
|---|---|---|---|---|---|---|---|---|---|---|
| Camel rep A | 8.7 | 1.3 | 1.2 | 7.1 | 7.9 | 7.6 | 7.3 | 7.1 | 6.6 | 6.6 |
| Camel rep B | 7.4 | 1.0 | 1.2 | 6.8 | 9.2 | 6.6 | 6.5 | 9.0 | 5.8 | 5.8 |
| Camel rep C | 8.4 | 2.5 | 2.0 | 7.3 | 6.9 | 7.9 | 7.3 | 7.6 | 7.1 | 7.3 |
| Calf rep A | 8.6 | 2.1 | 1.8 | 7.3 | 6.8 | 9.9 | 9.8 | 9.5 | 8.6 | 9.5 |
| Calf rep B | 8.4 | 1.0 | 1.3 | 6.2 | 7.6 | 9.2 | 9.1 | 9.2 | 8.5 | 8.8 |
| Calf rep C | 8.4 | 1.8 | 1.7 | 6.9 | 7.0 | 9.8 | 9.7 | 9.5 | 8.9 | 8.8 |
| LSD | 0.9 | 1.1 | 1.1 | 1.3 | 1.3 | 1.4 | 1.4 | 1.3 | 1.2 | 1.2 |

| | | | | | | | | | | |
|---|---|---|---|---|---|---|---|---|---|---|
| LSD - least significant difference Means in a column that differ by more than the LSD are different (p<0.05) Attributes were scored on a 15-point product-specific scale (Meilgaard et al., 1999). Cheese textures generally fall between 1 and 15 on this scale. Hfirmness - Hand firmness, Hspring - Hand springiness, Hrecovery - Hand rate of recovery, firm - firmness, frac - fracturability, degree brkdwn - degree of breakdown, coh - cohesiveness, adh - adhesiveness, smth mass - smoothness of mass, smthmthct - smoothness of mouthcoating. | | | | | | | | | | |

### Example 3:

### Cheese yield trials

Cheddar cheese (50+) was manufactured from 150 L of milk acidified with starter culture RST-630 (Chr. Hansen A/S, 0.01%) and coagulated with either CHY-MAX M (camel chymosin of the present invention, 2660 IMCU/100L) or CHY-MAX® (Chr. Hansen A/S, calf chymosin B, 3550 IMCU/100L). Camel chymosin dosage was reduced by 25% in IMCU compared to calf chymosin. On one day either two or three vats were produced. When three vats were produce one coagulant was made in duplicate. With a total of 12 days was made incl. 8 days doing duplicates (four for each coagulant) a total of 28 vats were produced.

**Table 3. Results of small-scale cheese yield trials calculated in percentage to reference (CHY-MAX ®).**

| **Difference to CHY-MAX** | **CHY-MAX (reference)** | **CHY-MAX M** |
|---|---|---|
| Moisture adjusted yield | 0 | +0.2% |
| Whey fat | 0 | -2.7% |
| Whey protein | 0 | -0.4% |

Cheeses produced with camel chymosin had in average a higher yield (+0.2%) compared to reference cheeses. Measurements on the whey composition supported this tendency with significant lower level fat (-2.7%) and lower protein (-0.4%) in whey of camel chymosin cheeses. This indicated higher cheese yield together with the reduced losses into whey demonstrates a clear tendency of camel chymosin being more efficient yield-wise compared to calf chymosin.

### Example 4:

Is it appears, camel chymosin has both a higher specific activity (measured as IMCU pr mg) and a faster action as the time to cutting is approx. 20% shorter than if bovine (calf) chymosin is used and the same enzymatic activity measured as IMCU is added to the milk.

In the following table, a measure for dosage of camel chymosin is calculated, ie the "X-factor", based on the data in example 1 (pH 6.5, 32 degrees C) and figures 1 and 1a.

| **Measure** | **Camel chymosin** | **Calf chymosin** | **Comments** |
|---|---|---|---|
| Specific activity (IMCU/mg) | 462 | 223 | 107% higher specific activity of camel chymosin. Earlier indicated 70% higher (ref D2: *Kappelar et al*.). |
| IMCU dosage (IMCU per liter) | 32.5 | 32.5 | Normal dosage level for cheese make |
| Time to clotting | 12-13 min | 12-13 min | Approx. same time from addition to clotting (flocculation), which correlates the to same IMCU dosed |
| Time to cutting firmness | 26-27 min | 32-33 min | 5-6 min faster curd formation or approx. 20% less time needed. |
| Calculation of X-factor | 32.5^{∗}26.5/1 = 860 | 32.5^{∗}32.5/1 = 1060 | IMCU dosage = (X^{∗}I)/min |

Suggested X-factor values for the dosage of camel chymosin (CHY-MAX® M) applied in different cheese types:

| Cheese type | Dosage (IM-CU/liter) | Volume of cheese milk (L) | Time until cutting (min) | X-factor X = (IMCU^{∗}min/L) |
|---|---|---|---|---|
| **Continental** | 30 | 1 | 30 | 900 |
| **Cheddar** | 35 | 1 | 30 | 1050 |
| **Pasta Filata** | 28 | 1 | 30 | 840 |
| **Soft cheese** | 24 | 1 | 30 | 720 |

Suggested X-factor values for the dosage of camel chymosin (CHY-MAX® M) at different pH value at addition:

| pH of cheese milk | Dosage (IM-CU/liter) | Volume of cheese milk (L) | Time until cutting (min) | X-factor X = (IMCU^{∗}min/L) |
|---|---|---|---|---|
| **6.7** | 45 | 1 | 30 | 1350 |
| **6.6** | 37 | 1 | 30 | 1110 |
| **6.5** | 31 | 1 | 30 | 930 |
| **6.4** | 26 | 1 | 30 | 780 |
| **6.3** | 22 | 1 | 30 | 660 |

In accordance with these data the amount of camel chymosin can be calculated for various cheese types and various pH values of the cheese milk. The skilled person can without any inventive effort calculate the amount of camel or tylopoda chymosin to be added to milk. Thus, the invention relates to the following methods for manufacturing cheese (in which "L" means liters of milk, and "desired time for coagulation" is the time period from addition of chymosin to cutting, measured in minutes), and to the cheeses produced by any method:

A method for manufacturing a continental type cheese comprising contacting bovine milk with *Tylopoda* chymosin at a dosage below 1100 (such as below 900 or 800) IMCU ^{∗} L / desired time for coagulation.

A method for manufacturing a cheddar type cheese comprising contacting bovine milk with *Tylopoda* chymosin at a dosage below 1300 (such as below 1100 or 1000) IMCU ^{∗} L / desired time for coagulation.

A method for manufacturing a pasta filata type cheese comprising contacting bovine milk with *Tylopoda* chymosin at a dosage below 900 (such as below 800 or 700) IMCU ^{∗} L / desired time for coagulation.

A method for manufacturing a soft cheese comprising contacting bovine milk with *Tylopoda* chymosin at a dosage below 800 (such as below 700 or 600) IMCU ^{∗} L / desired time for coagulation.

A method for manufacturing a cheese comprising
- contacting bovine milk at a pH in the range 6.2-6.4 with *Tylopoda* chymosin at a dosage below 850 (such as below 750 or 700) IMCU ^{∗} L / desired time for coagulation; or
- contacting bovine milk at a pH in the range 6.3-6.5 with *Tylopoda* chymosin at a dosage below 1000 (such as below 900 or 800) IMCU ^{∗} L / desired time for coagulation; or
- contacting bovine milk at a pH in the range 6.4-6.6 with *Tylopoda* chymosin at a dosage below 1200 (such as below 1100 or 1000) IMCU ^{∗} L / desired time for coagulation; or
- contacting bovine milk at a pH in the range 6.5-6.7 with *Tylopoda* chymosin at a dosage below 1450 (such as below 1350 or 1200) IMCU ^{∗} L / desired time for coagulation; or
- contacting bovine milk at a pH in the range 6.6-6.8 with *Tylopoda* chymosin at a dosage below 1600 (such as below 1500 or 1400) IMCU ^{∗} L / desired time for coagulation.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

### REFERENCES

Biochemical and Biophysical Research Communications Volume 342, Issue 2, 7 April 2006, Pages 647-654
WO02/36752A2
WO02/50253A
US patent application 10/807115, filed 24 March 2004
US patent application 09/705917, filed 06 November 2000
Journal of Dairy Research (2000) 67 73-81
Milchwissenschaft (1993) 48, 322
Brown, J.A., Foegeding, E.A., Daubert, C.R., Drake, M.A., and Gompertz, M. 2003. Relationships among rheological and sensorial properties of young cheeses. J. Dairy Sci. 86, 3054-3067.
Drake, M.A., McIngvale, S.C., Cadwallader, K.R., and Civille, G.V. 2001. Development of a descriptive sensory language for Cheddar cheese. J. Food Sci. 66:1422-1427.
Drake, M.A., Keziah, M.D., Gerard, P.D., Delahunty, C.M. Sheehan, C., Turnbull, R.P. and Dodds, T.M., 2005. Comparison of differences between lexicons for descriptive analysis of Cheddar cheese flavor in Ireland, New Zealand, and the United States. Int. Dairy J. 15:473-483.
Meilgaard, M.M., Civille, G.V., and B.T. Carr. 1999. Selection and training of panel members. Pages 174-176 in Sensory Evaluation Techniques, 3nd ed. CRC Press, Boca Raton, FI.
International IDF standard 157A:1997, International Dairy Federation, 41 Square Vergote, 1030 Brussels, Belgium.

All references cited in this patent document are hereby incorporated herein in their entirety by reference.

The following represent further embodiments of the present invention:
1. A method for manufacturing a dairy product (e.g. cheese or curd), comprising contacting bovine milk with a chymosin enzyme having an amino acid sequence identical or substantially identical to the amino acid sequence of chymosin (EC 3.4.23.4) from an animal of the suborder *Tylopoda,* said chymosin is added in an amount not exceeding 30 IMCU per liter milk and/or said chymosin is added in an amount not exceeding 90% of the amount (measured in IMCU) of bovine chymosin B that would have been necessary for obtaining a curd with the same strength (firmness) in the same time and at the same temperature using the same milk.
2. A method for manufacturing a dairy product (e.g. cheese or curd) comprising obtaining a curd (such as with a firmness of 20mm +/- 5mm measured on Formagraph equipment) by contacting bovine milk with *Tylopoda* chymosin at a concentration (amount) below 950 IMCU multiplied by volume of milk in liter and divided by desired time for coagulation (cutting time, i.e. time from addition of chymosin to cutting) in minutes (i.e. calculated as 950 x L / minutes).
3. A method for improving taste and/or texture of a dairy product (e.g. cheese or curd), comprising contacting bovine milk with a chymosin enzyme having an amino acid sequence identical or substantially identical to the amino acid sequence of chymosin (EC 3.4.23.4) from an animal of the suborder *Tylopoda.*
4. The method according to the preceding embodiments, wherein the dairy product is cheese, e.g. a continental type cheese, a pasta filata type cheese, cheddar type cheese, mascarpone, feta, soft cheese, brie, camembert, fresh cheese, or cottage cheese.
5. The method according to any preceding embodiment, wherein the milk has a pH in the range of 6.0 to 7.0, in the range 6.2 to 6.8, or in the range 6.4 to 6.6.
6. The method according to any preceding embodiment, wherein the milk has a temperature within the range 25-37 degrees C.
7. The method of any preceding embodiment, wherein the bovine milk is from an animal species selected from the group consisting of: cow, buffalo, sheep and goat; or the milk is a composition which comprises milk from at least of one animal species selected from the group consisting of: cow, buffalo, sheep and goat.
8. The method of any preceding embodiment, wherein the bovine milk is cow's milk; or the milk is a composition which comprises cow's milk.
9. The method of any preceding embodiment, wherein the chymosin enzyme is used in an amount below 6.5 mg per 100 liter of milk.
10. The method of any preceding embodiment, wherein the chymosin is used in the ratio from 5 to 25 IMCU per liter of milk.
11. The method of any preceding embodiment, wherein the time for curd formation is in the range of 10 to 60 minutes.
12. The method of any preceding embodiment, wherein the animal of the suborder *Tylopoda* is an animal belonging to the family *Camelidae.*
13. The method of the preceding embodiment, wherein the animal belongs to a species selected from the group consisting of *Camelus dromedarius, Camelus bactrianus* and *Lama glama.*
14. The method of any preceding embodiment, wherein the chymosin has a sequence identity of at least 90% to the amino acid sequence 59-381 of any of the sequences: SEQ ID No:1, SEQ ID No:2, or SEQ ID No:3.
15. The method of any preceding embodiment, wherein the chymosin has a sequence identity of at least 90% (preferable at least 95%, more preferable at least 98% or most preferable at least 99%) to the amino acid sequence 59-381 of SEQ ID No:1.
16. The method of any preceding embodiment, wherein the chymosin contains an amino acid sequence which has a sequence identity of at least 95% (preferable at least 96%, more preferable at least 98% or most preferable at least 99%) to any 50 aa length fragments of the sequence 59-381 of SEQ ID No: 1.
17. The method of any preceding embodiment, wherein the chymosin contains an amino acid sequence selected from the group consisting of: SEQ ID No:5, SEQ ID No:6, and SEQ ID No:7.
18. The method of any preceding embodiment, wherein the chymosin has the amino acid sequence 59-381 of SEQ ID No: 1.
19. The method of any preceding embodiments, wherein the chymosin is produced using a bacteria, a yeast; or a fungus (including a filamentous fungus) as host organism.
20. The method of the preceding embodiment, wherein the fungus is an *Aspergillus* species, such as *Aspergillus niger.*
21. The method of any preceding embodiments, wherein a further enzyme, e.g. a kappa-casein cleaving enzyme, is contacted with the milk.
22. The method of the preceding embodiment, wherein the further enzyme is used in an amount of up to 50%, measured in IMCU, of the *Tylopoda* chymosin.
23. The method of any preceding embodiments, wherein the milk is not contacted with a camel pepsin (EC 3.4.23.1, especially the pepsin obtainable from camel calf abomasum) in an amount exceeding 30%, measured in IMCU, of the chymosin.
24. The method of any preceding embodiment, wherein the milk is not contacted with camel pepsin, especially the pepsin obtainable from camel calf abomasum.
25. The method of any preceding embodiments, wherein the milk is tempered (before or after contacting with chymosin) to a temperature in the range 20 to 50 degrees C.
26. A method for manufacturing cheese comprising contacting bovine milk with a chymosin enzyme having an amino acid sequence identical or substantially identical to the amino acid sequence of chymosin (EC 3.4.23.4) from an animal of the suborder *Tylopoda.*
27. A method for manufacturing cheese comprising contacting bovine milk with a chymosin enzyme having an amino acid sequence identical or substantially identical to the amino acid sequence of chymosin (EC 3.4.23.4) from an animal of the suborder *Tylopoda,* provided that said chymosin is not identical to SEQ ID No 1, amino acids 59-381.
28. A chymosin enzyme having an amino acid sequence identical to or having a sequence identity of at least 90% to the amino acid sequence of chymosin (EC 3.4.23.4) from an animal of the suborder *Tylopoda* or to one or more of the following sequences: SEQ ID No: 1; SEQ ID No:2; SEQ ID No:3; and SEQ ID No:4.
29. A dairy product obtainable by a method according to any of embodiments 1 to 27.
30. A DNA sequence encoding a *Tylopoda* chymosin enzyme, i.e. a sequence that is substantially identical to one of the following sequences: SEQ ID No:8; SEQ ID No:9; SEQ ID No:10; or SEQ ID No: 11; and/or is able to hybridize to one of the complementary sequences thereto under stringent conditions.

## Claims

1. A method for manufacturing a dairy product comprising obtaining a curd (such as with a firmness of 20mm +/- 5mm measured on Formagraph equipment) by contacting bovine milk with *Tylopoda* chymosin (EC 3.4.23.4) at a concentration (amount) below 950 IMCU multiplied by volume of milk in liter and divided by desired time for coagulation (cutting time, i.e. time from addition of chymosin to cutting) in minutes.

2. A method for improving taste and/or texture of cheese, comprising contacting bovine milk with a chymosin enzyme having an amino acid sequence identical to the amino acid sequence of chymosin (EC 3.4.23.4) from an animal of the suborder *Tylopoda.*

3. The method according to claim 1 or 2, comprising contacting bovine milk with a chymosin enzyme having an amino acid sequence identical to the amino acid sequence of chymosin (EC 3.4.23.4) from an animal of the suborder *Tylopoda,* wherein said chymosin is added in an amount not exceeding 30 IMCU per liter milk.

4. The method according to any of the preceding claims, wherein the chymosin is used in the ratio from 5 to 25 IMCU per liter of milk.

5. The method according to any of the preceding claims, wherein the cheese is a continental type cheese, a pasta filata type cheese, cheddar type cheese, mascarpone, feta, soft cheese, brie, camembert, fresh cheese, or cottage cheese.

6. The method according to any of the preceding claims, wherein the milk has a temperature within the range 25-37 degrees C.

7. The method according to any of the preceding claims, wherein the chymosin enzyme is used in an amount below 6.5 mg per 100 liter of milk.

8. The method according to any of the preceding claims, wherein the chymosin has a sequence identity of at least 90% to the amino acid sequence 59-381 of any of the sequences: SEQ ID No:1, SEQ ID No:2, or SEQ ID No:3.

9. The method according to any of the preceding claims, wherein the chymosin has the amino acid sequence 59-381 of SEQ ID No:1.

10. The method according to any of the preceding claims, wherein the chymosin is produced using a bacteria, a yeast; or a fungus (including a filamentous fungus) as host organism.

11. The method according to any of the preceding claims, wherein the fungus is an *Aspergillus* species, such as *Aspergillus niger.*

12. The method according to any of the preceding claims, wherein a further enzyme, e.g. a kappa-casein cleaving enzyme, is contacted with the milk.

13. The method according to any of the preceding claims, wherein the further enzyme is used in an amount of up to 50%, measured in IMCU, of the *Tylopoda* chymosin.

14. The method according to any of the preceding claims, wherein the milk is not contacted with a camel pepsin (EC 3.4.23.1, especially the pepsin obtainable from camel calf abomasum) in an amount exceeding 30%, measured in IMCU, of the chymosin.

15. A dairy product obtainable by a method according to any of claims 1 to 14.
